# EUROPÄISCHE PATENTSCHRIFT

(11) **EP 1 599 505 B1**
(45) Veröffentlichungstag und Bekanntmachung des Hinweises auf die Patenterteilung: **05.08.2009**
(21) Anmeldenummer: 04717082.4
(22) Anmeldetag: 04.03.2004
(51) Int. Cl.: C08B 37/00, C08B 37/18, C12P 19/04

(54) **VERFAHREN ZUR HERSTELLUNG EINES BETA-1,3-GLUKANS MIT VERBESSERTEN EIGENSCHAFTEN**
METHOD FOR PRODUCING A BETA-1,3-GLUCAN WITH IMPROVED CHARACTERISTICS
PROCEDE DE PRODUCTION D'UN BETA-1,3-GLYCANE A PROPRIETES AMELIOREES

(30) Priorität: 04.03.2003 DE 10309281
(43) Veröffentlichungstag der Anmeldung: 30.11.2005
(73) Patentinhaber: SATIA GMBH, 85354 Freising (DE)
(72) Erfinder: FROHNWIESER, Werner, 86567 Hilgertshausen (DE); VOLLAND, Michael, 85757 Karlsfeld (DE); WITTMANN, Evi, 83301 Traunreut (DE); SKORUPINSKI Fabienne, F-50200 Bricqueville la Blouette (FR); LEBEHOT, Jean-Jacques, F-50500 Saint Pellerin (FR); LEMOIGNE, Yves, F-50700 Lieusaint (FR); LÖTZBEYER, Thomas, 85386 Eching (DE)
(74) Vertreter: Böhm, Brigitte
(86) Internationale Anmeldenummer: PCT/EP2004/002203
(87) Internationale Veröffentlichungsnummer: WO 2004/078788

(56) Entgegenhaltungen:
- EP-A- 0 039 962
- WO-A-90/04334
- B. J. CATLEY UND M. E. FRASER: "Susceptibility of Scleroglucan to the (1->3)-beta-D-Glucanase Zymolase" CARBOHYDRATE RESEARCH, Bd. 183, Nr. 1, 1988, Seiten 83-88, XP002280067 Amsterdam/NL

## Beschreibung

Gegenstand der vorliegenden Erfindung ist ein Verfahren zur Herstellung eines β-1,3-Glukans, spezielle β-1,3-Glukane, eine feste Formulierung sowie die Verwendung der speziell hergestellten β-1,3-Glukane.

Bei β-1,3-Glukanen, zu denen u.a. auch die Skleroglukane gerechnet werden, handelt es sich um entsprechend polysaccharidisch verknüpfte Glukosemoleküle.

Skleroglukane sind wasserlösliche, nicht-ionische natürliche Polymere, die durch zahlreiche filamentäre Pilze, wie bspw. Sclerotium rolfsii, produziert werden. Im industriellen Maßstab erhält man Skleroglukane durch aerobe, submerse Kulturen ausgewählter Stämme. Skleroglukane bestehen aus β-1,3-D-Glucose-Molekülen und weisen an jedem dritten Zuckermolekül β-1,6-D-Glucose-Seitenketten auf. Das durchschnittliche Molekulargewicht ist > 10⁶ Da.

Skleroglukan als technisches Polymer wird hauptsächlich bei der Erdölförderung zur Eindickung des Bohrschlammes eingesetzt. Ebenso gebräuchlich ist aber dessen Verwendung in Klebern, Wasserfarben, Druckertinten, Kosmetika und in der pharmazeutischen Industrie. Dieses Biopolymer bildet in Wasser pseudoplastische Lösungen mit scherverdünnenden Eigenschaften und es toleriert zudem hohe Temperaturen, breite pH-Bereiche und ist auch gegenüber Elektrolyten beständig.

Die meisten Skleroglukane werden auf wirtschaftliche Weise hergestellt, indem sie aus der Fermentationsbrühe präzipitiert und als Feststoff gewonnen werden. Aufgrund ihrer Viskositätseigenschaften ist es im allgemeinen nicht möglich, sämtliche während der Fermentation freigesetzten Feststoffe vor dem Präzipitationsschritt abzutrennen, so dass die getrockneten und festen Skleroglukane normalerweise gewisse Anteile an wasserunlöslichen Feststoffen in Form von Zellbruchstücken aufweisen. Diese Feststoffe wiederum bleiben beim Auflösen der Skleroglukane in Wasser ungelöst, was zur Folge hat, dass die Skleroglukane für Anwendungsfälle, in denen bestimmte Reinheitsgrade der Polysaccharid erforderlich sind, zusätzlich aufgereinigt werden müssen. Dazu werden die Fermentationsbrühen aufgrund der erhöhten Viskosität zunächst verdünnt und dann in Vorbereitung des sich anschließenden Filtrationsschritts mit einem Filtrierhilfsmittel versetzt. Diese Vorgehensweise ist sehr zeit- und energieintensiv und der Ertrag an aufgereinigtem Polysaccharid ist mit < 50 % relativ gering. Nachteilig dabei ist zusätzlich die keinesfalls zufriedenstellende Trübheit der resultierenden Skleroglukan-Lösung.

Zur Umgehung dieser Nachteile wurden zahlreiche Verfahren entwickelt, wie z.B. das Verfahren gemäß US 4,165,257, das die Zugabe eines kaustischen Enzyms, wie der Esperase, zum Abbau proteinähnlicher Zellbruchstücke beschreibt. Zusätzlich offenbart US 4,119,491 die Zugabe fester, silikatischer Materialien, wodurch eine Klärung der Polysaccharide ohne substantiellen Viskositätsverlust erzielt wird. DE-OS 195 47 748 beschreibt die Zugabe eines Detergens zur Fermentationsbrühe, was zu einer Phasentrennung führt und die Polysaccharide in der Kopfphase anreichert.

Ein mechanisches Verfahren zur Aufreinigung Polysaccharid-haltiger Lösungen wird in EP-A 514 890 vorgeschlagen: Dabei wird eine wässrige Lösung der Polysaccharide mit Hilfe eines Rührgerätes mit einem hydrophilen organischen Lösemittel vermischt, wodurch das Polysaccharid jedoch nicht gelöst wird.

Die Dokumente DE-OS 3835771, US 4,299,825 und US 3,355,447 beschreiben jeweils Verfahren zur Verbesserung der Filtrierbarkeit von Polysaccharid-haltigen Lösungen durch Hitzebehandlung, Filtration oder Ultrafiltration. Zur Gewinnung einer konzentrierten Xanthan-Lösung wird gemäß EP-PS 049 012 eine Ultrafiltration auch in Verbindung mit einer enzymatischen Behandlung vorgeschlagen.

Entsprechend der EP-PS 039 962 wird zum Abbau wasserunlöslicher Bestandteile in wässrigen Polysaccharid-haltigen Lösungen aus der Fermentation von Xanthomonas die Verwendung eines Enzymkomplexes mit zelllytischen β(1,3)-Glucanase- und Protease-Aktivitäten aus Pellicularia sp. empfohlen.

Gemäß US 4,416,990 ist ein enzymatisches Klärungsverfahren für unreinen Xanthan Gum geschützt, der mindestens bakterielle Zellbestandteile oder Mikrogele enthält, indem eine Polysaccharase-Zubereitung von Basidomycetes polyporaceae-Cellulase zugesetzt wird. Eine enzymatische Klärung eines natürlichen Xanthan-Harzes in wässriger Phase beschreibt DE-OS 3 139 249: Zur Entfernung bakterieller Zellreste oder Mikrogele wird in diesem Fall eine Cellulase von Basidomycetes sp. eingesetzt.

Alle beschriebenen Neuerungen haben jeweils eine spezielle Verbesserung zum Ziel, wobei die einzelnen Verbesserungen im wesentlichen auf zwei Haupteigenschaften von Polysaccharid-Lösungen abzielen:

Zum einen soll die Klarheit entsprechender Lösungen verbessert werden und zum anderen soll die Filtration erleichtert und das Filtrationsergebnis verbessert werden.

Bekannt sind auch zahlreiche Verfahren, mit denen Glukan-haltige Zellbestandteile mit β-1,3-Glucanasen oder mit Enzymen entsprechender Aktivität behandelt werden.

So ist aus EP-PS 440 725 die Bereitung eines Glukans aus Saccharomyces cerevisiae bekannt, bei dem auf eine Endo-β-Glucanase in Form von Laminarinase zurückgegriffen wird.

US 6,090,615 beschreibt ein Verfahren zur Herstellung eines β-Glukan-haltigen Extraktes aus einem ein Mycel enthaltenden Kulturmedium mit Hilfe von β-1,3-Glucanasen. Hierbei wird die Glucanase allerdings nicht alleine sondern in Kombination mit Chitinase und Cellulase so eingesetzt, dass aus dem Mycel als Ausgangsmaterial durch einen Aufschluss die im Mycel enthaltenen Inhaltsstoffe freigesetzt werden.

Aus den US-Patenten 5,250,436 und 4,810,646 sind jeweils Verfahren zur Gewinnung von Glukan durch den Abbau von Glukan-haltigen Matrices mit Laminarinase vorbeschrieben. Dabei wird aus Hefezellen durch eine Alkali- und eine nachfolgende Säurebehandlung die Bindungsstruktur der darin enthaltenen Glukane verändert, wodurch diese in Abhängigkeit vom eingesetzten Hefestamm typische Viskositätseigenschaften entfalten.

Die Gewinnung von Mikrokapseln steht im Vordergrund von US 5,521,089. Bei diesem Verfahren werden Hefezellen mit einer β-1,3-Glucanase behandelt, wodurch Mikrokapseln erhalten werden, die zum Einschluss hydrophober Flüssigkeiten geeignet sind.

Die Modifikation von β-Glukanen, wie bspw. Curdlan oder Laminarin, wird entsprechend US 6,284,509 durch den Einsatz von β-1,3-Glucanasen erreicht.

WO 90/04334 offenbart Zusammensetzungen, die zur Behandlung von diätetischen Erkrankungen geeignet sind, sowie diätetische Zusatzmittel, um Ballaststoffe und kurzkettige Fettsäuren bereitzustellen und um den Serum-Cholesterinspiegel zu vermindern. Weiterhin offenbart WO 90/04334 Quellstoffe für Menschen und Tiere sowie Verfahren zu deren Verwendung. Die Zusammensetzungen und Verfahren beruhen auf β-Glukanen.

B. J. Catley und M. E. Fraser, Carbohydrate Res., 183 (1988), 83-88 beschreibt die Empfindlichkeit von Scleroglukan gegenüber der 1,3-β-D-Glukanase Zymolase. Insbesondere wird die Behandlung eines Scleroglukans und eines Curdlans mittels einer 1,3-β-D-Glukanase mit und ohne vorherige NAOH-Behandlung verglichen.

EP 0 039 962 offenbart ein verbessertes Verfahren für den Abbau von unlöslichen Materialien, die mittels einer Fermentation von Kohlenhydraten mit Xanthomonas in wässrigen Polysaccharidlösungen hergestellt werden, also ein verbessertes Verfahren zum Abbau des Produktes Xanthan, welches bei der Xanthomonas-Fermentation entsteht.

Insgesamt fällt auf, dass die mit den beschriebenen Verfahren erzielten Ergebnisse zu keinen gleichzeitig verbesserten rheologischen Eigenschaften, gesteigerten Löslichkeiten und erhöhten Filtrationsausbeuten führen.

Aus den geschilderten Nachteilen des Standes der Technik hat sich für die vorliegende Erfindung die Aufgabe gestellt, ein Verfahren zur Herstellung eines β-1,3-Glukans bereitzustellen, mit dem Glukane erhalten werden, die nach Möglichkeit eine verbesserte Kaltwasserlöslichkeit, eine gesteigerte Viskosität und verringerte Trübheit sowie deutlich verringerte Anteile an unlöslichen Bestandteilen aufweisen und mit dem eine deutlich verbesserte Filtrierbarkeit verbunden ist.

Gelöst wurde diese Aufgabe mit einem entsprechenden Verfahren zur Herstellung eines β-1,3-Glukans, bei dem eine Glukan-haltige Matrix in Wasser als Lösemittel gerührt und anschließend bei Temperaturen zwischen 15 und 60°C sowie bei einem pH-Wert zwischen 4,0 und 10,0 mit einem Protein mit β(1,3)-Glucanase-Aktivität behandelt wird.

Überraschend wurde gefunden, dass mit diesem Verfahren die in unlöslicher Form an das Mycel gebundenen Glukan-Moleküle aufgrund enzymatischer Aktivitäten in wässrige Lösungen freigesetzt werden können. Entsprechend der Aufgabenstellung gelingt es mit diesem Verfahren auch, die Viskosität von Glukan-haltigen Lösungen zu steigern und zusätzlich die Anteile an unlöslichen Bestandteilen zu verringern sowie die Kaltwasserlöslichkeit der Glukane deutlich zu verbessern.

Als besonders geeignet im Sinn der vorliegenden Erfindung haben sich Matrices erwiesen, deren β-1,3-Glukane β(1,6)-Glucose-Seitenketten aufweisen.

Als bevorzugte Variante kann auch ein Verfahren angesehen werden, bei dem als Protein eine β(1,3)-Glucanase eingesetzt wird und insbesondere ein Protein, das zusätzlich zur β(1,3)-Glucanase-Aktivität auch eine β(1,4)-Glucanase-Aktivität zeigt. β(1,3)-Glucanasen, wie sie von der vorliegenden Erfindung vorzugsweise benutzt werden, werden von unterschiedlichen Mikroorganismen hergestellt, wie z.B. Trichoderma oder Bacillus.

Bezüglich der Konzentration des Proteins mit β(1,3)-Glucanase-Aktivität hat es sich im Zusammenhang mit der vorliegenden Erfindung als empfehlenswert erwiesen, wenn diese zwischen 0,001 und 3,0 Gew.-% beträgt, insbesondere 0,01 bis 1,0 Gew.-% und besonders bevorzugt 0,1 bis 0,5 Gew.-%, jeweils bezogen auf die Reaktionsmischung.

In Abhängigkeit vom gewählten Protein bzw. Enzym und/oder dessen Konzentration sieht die vorliegende Erfindung Reaktionstemperaturen vor, die zwischen 15 und 60 °C und vorzugsweise zwischen 20 und 40 °C liegen, wobei Raumtemperatur als besonders bevorzugt anzusehen ist.

Im Rahmen der vorliegenden Erfindung werden als bevorzugte Glukan-haltige Matrices Fermentationsbrühen, Kulturmedien und Suspensionen eingesetzt, aber auch Mycele, Hydrokolloide oder Pulver-Zubereitungen, die einen Lösemittel-Anteil von 20 bis 99,9 Gew.-% und insbesondere von 50 bis 99 Gew.-%, jeweils bezogen auf den Feststoff-Gehalt, aufweisen. Bspw. können die wasserunlöslichen und das Glukan-enthaltenden Mycele in Form fester Zusammensetzungen mit dem Enzym-Komplex behandelt werden, wobei diese festen Zusammensetzungen besonders dadurch vorteilhaft erscheinen, als sie in einer Ein-Stufen-Reaktion umgesetzt werden können.

Die vorliegende Erfindung sieht für das Verfahren die bevorzugte Verwendung von Fermentationsbrühen oder Kulturmedien vor, die ungelöste Feststoffe, Zellbestandteile und/oder Zellbruchstücke enthalten. Gleichermaßen besonders gut geeignet sind aber auch Mycele, Hydrokolloide oder Pulver-Zubereitungen, die als wässrige Lösungen eingesetzt werden.

Üblicherweise handelt es sich beim erfindungsgemäß eingesetzten Polysaccharid um ein hydrophiles Kolloid, das durch Fermentation in einem gewöhnlichen Nährmedium mit Hilfe von Mikroorganismen erhalten werden. Derartige Glukane, bspw. in Form von Skleroglukanen, und ihre Zubereitungen, können in Form der Fermentationsbrühen oder des Kulturmedium verwendet werden, wobei sie wie beschrieben ungelöste Feststoffe und Zellbestandteile oder Zellbruchstücke enthalten können.

Das Verfahren gemäß der Erfindung wird durchgeführt, indem das Protein mit enzymatischer Aktivität einer das Polysaccharid enthaltenden Matrix in Form einer wässrigen Lösung, welche zusätzlich die unlöslichen Bestandteile enthält, zugesetzt wird und anschließend diese Mischung stehen gelassen wird. Ein entscheidendes Kriterium für die Dauer und den Erfolg der Reaktion ist der Zeitbedarf für die enzymatisch bedingte Freisetzung der an das Mycel gebundenen Polysaccharide in die Lösung. Normalerweise ist es vollkommen ausreichend, wenn die wässrige Lösung 0,03 bis 3,0 Gew.-% des Polysaccharids enthält. Die Konzentration des verwendeten Proteins mit Enzym-Aktivität hängt immer direkt von der Glukan-Konzentration ab und der Menge der darin enthaltenen unlöslichen Zellbestandteile.

Wie bereits angedeutet, können die eingesetzten Matrices in Form eines Mycels, eines Hydrokolloids oder einer Pulver-Zubereitung auch bestimmte Lösemittel-Anteile aufweisen oder als wässrige Lösungen eingesetzt werden, wobei erfindungsgemäß Wasser als Lösemittel für die Matrix verwendet wird.

Zur Erzielung eines optimalen enzymatischen Umsatzes werden bei dem erfindungsgemäßen Verfahren die eingesetzten Matrices gerührt, um so ein Absetzen der Feststoffe zu vermeiden und eine vergleichmäßigte Konzentration der eingesetzten Proteine mit enzymatischer Aktivität in der Polysaccharid-haltigen Lösung zu gewährleisten.

Insgesamt haben sich Proteine mit β(1,3)-Glucanase-Aktivität und vor allem β(1,3)-Glucanasen als durchaus pH-tolerant gezeigt, jedoch werden für das vorliegende Verfahren pH-Werte der Matrices empfohlen, die zwischen 4,0 und 10,0 und insbesondere zwischen 5,0 und 7,0 liegen. Unter den genannten Bedingungen kann eine maximale Freisetzung der Glukane aus dem unlöslichen Zellmaterial innerhalb relativ kurzer Zeiträume gewährleistet werden. Aus diesem Grund beansprucht die Erfindung als Dauer für die enzymatische Behandlung Zeiträume zwischen 15 Minuten und 24 Stunden und insbesondere 1 bis 6 Stunden.

Das beanspruchte Verfahren kann zwar auch batchweise durchgeführt werden, jedoch wird ein kontinuierliches Verfahren bevorzugt, wobei das Protein mit enzymatischer Aktivität einem Vorlagebehälter mit dem wässrigen Polysaccharid in Form einer verdünnten oder unverdünnten Fermentationsbrühe oder einer wässrigen Lösung des isolierten Glukans zugesetzt wird. Besonders bei kontinuierlicher Fahrweise wird empfohlen, das Reaktionsgefäß oder den Behälter in einer ausreichenden Größe zu wählen und die Zugaberate des Enzyms und des Polysaccharids so festzulegen, dass der wässrigen Polysaccharid-Lösung mit den festen Zellbestandteilen in Gegenwart einer ausreichenden Enzymkonzentration genügend Zeit zum gewünschten Zellabbau und zur Freisetzung der Polysaccharide zur Verfügung steht.

Von der vorliegenden Erfindung ist auch eine Verfahrensvariante umfasst, bei der die Glukan-haltige Matrix nach erfolgter enzymatischer Behandlung einer Wärmebehandlung bei Temperaturen zwischen 70 und 150 °C und insbesondere zwischen 80 und 140 °C unterzogen wird. Dabei sollte die Wärmebehandlung 1 bis 60 Minuten lang und insbesondere für 2 bis 30 Minuten durchgeführt werden. Diese Hitzebehandlung dient insbesondere dazu, Mikroorganismen bzw. enzymatisch aktive Proteine zu inaktivieren.

Abschließend können die Glukan-haltigen Matrices einer Filtration und/oder Zentrifugation unterzogen werden, was die vorliegende Erfindung ebenfalls vorsieht. Der Filtrationsvorgang kann bspw. mit Hilfe einer Filterpresse und gegebenenfalls unter Einsatz eines Filtrierhilfsmittels durchgeführt werden, wobei in jedem Falle als Produkt ein gereinigtes Glukan erhalten wird.

Zur Vervollständigung des beanspruchten Verfahrens kann aus der enzymbehandelten und gegebenenfalls filtrierten Glukan-haltigen Lösung gemäß vorliegender Erfindung das Glukan abgetrennt werden, was insbesondere durch Evaporation, Gefriertrocknung oder Ausfällung erfolgen sollte. Im Falle einer Evaporation wird das Wasser durch Erhitzen entfernt; die Ausfällung des Glukans kann durch Zusatz von Alkoholen erfolgen, die Entfernung von Lösemittel(-resten) durch Filtration. Für eine erfolgreiche Evaporation des Wassers durch Hitze wird ein Temperaturbereich zwischen 80 und 100 °C vorgeschlagen; für die Gefriertrocknung eine Temperatur von 20 °C und ein Druck von 0,01 hPa. Sollte eine Präzipitationsschritt durchgeführt werden, so wird die Polysaccharid-haltige Lösung in reinen Alkohol gegeben. Das Präzipitat wird anschließend durch Filtration mit einem Filtersieb entfernt und der abgetrennte Feststoff bei Raumtemperatur (ca. 25 °C) getrocknet.

Neben dem Verfahren zur Herstellung eines β-1,3-Glukans beansprucht die vorliegende Erfindung auch ein mit diesem Verfahren hergestelltes β-1,3-Glukan und insbesondere ein entsprechendes Glukan mit verbesserter Kaltwasserlöslichkeit und/oder verringerten Anteilen an unlöslichen Bestandteilen und/oder einer gesteigerten Viskosität und/oder einer verringerten Trübheit in wässrigen Lösungen und/oder einer verbesserten Filtrierbarkeit.

Gegenstand der vorliegenden Erfindung ist aber auch eine feste Formulierung, die mindestens 90 bis 99,9 Gew.-% eines unbehandelten β-1,3-Glukans enthält sowie 0,005 bis 0,1 Gew.-% eines Proteins mit β(1,3)-Glucanase-Aktivität sowie 0 bis 10 Gew.-% mindestens eines weiteren Inhaltstoffes, wie Füllstoffe, inerte Verdünnungsmittel oder ein Mycel. Dabei kann das eingesetzte β-1,3-Glukan wiederum β(1,6)-Glucose-Seitenketten aufweisen und das verwendete Protein zusätzlich eine β(1,4)-Glucanase-Aktivität zeigen. Diese Formulierungen können in fester Form direkt in Wasser oder andere wässrige Medien eingeführt werden, wobei sie den Vorteil besitzen, dass Enzyme und Polysaccharide nicht getrennt zugesetzt werden müssen.

Schließlich beansprucht die vorliegende Erfindung auch noch die Verwendung eines mit dem beschriebenen Herstellungsverfahren erhaltenen β-1,3-Glukans für kosmetische Anwendungen und/oder in der Körper- und Gesundheitspflege und/oder in der Nahrungs- und Lebensmittelindustrie und/oder in der Erdölförderung.

Zusammenfassend ist festzuhalten, dass die vorliegende Erfindung ein verbessertes Verfahren zur Herstellung von β-1,3-Glukanen zur Verfügung stellt, wobei die Ausbeuten deutlich höher ausfallen und die Qualität der damit erhaltenen Glukane und insbesondere der Skleroglukane durch eine enzymatische Behandlung der rohen Fermentationsbrühen oder des Glukanpulvers deutlich verbessert wird. Außerdem ist es mit diesem Verfahren möglich, durch eine enzymatische Behandlung unlösliche Mycel-Bestandteile durch Freisetzen Mycel-gebundener Polysaccharide zu verflüssigen, woraus eine erhöhte Viskosität der Polysaccharid-haltigen Lösungen resultiert.

Die nachfolgenden Beispiele verdeutlichen die angesprochenen Vorteile des beanspruchten Verfahrens zur Herstellung von β-1,3-Glukan und damit erhaltener Glukane.

### Beispiele

### Beispiel 1: Erhöhung der Viskosität einer Skleroglukan-haltigen Lösung

Zu 100 mL destillierten Wassers wurde 1 g Skleroglukan (Actigum CS6, Degussa AG) gegeben und mit einem Schraubenrührer 24 Stunden lang bei 20 °C gerührt. Anschließend wurden 10 mL dieser Skleroglukan-haltigen Lösung in ein Thermo Haake-Viskosimeter (Rotovisco C1) bei 37 °C und mit einer Scherrate von 10/Sekunde gegeben. Dann wurde 1 mL einer Lösung, die als Enzym 1,53 mg/mL destillierten Wassers einer Endo-β(1,3)-Glucanase (Megazyme) enthielt, gegeben und die Messung begonnen.

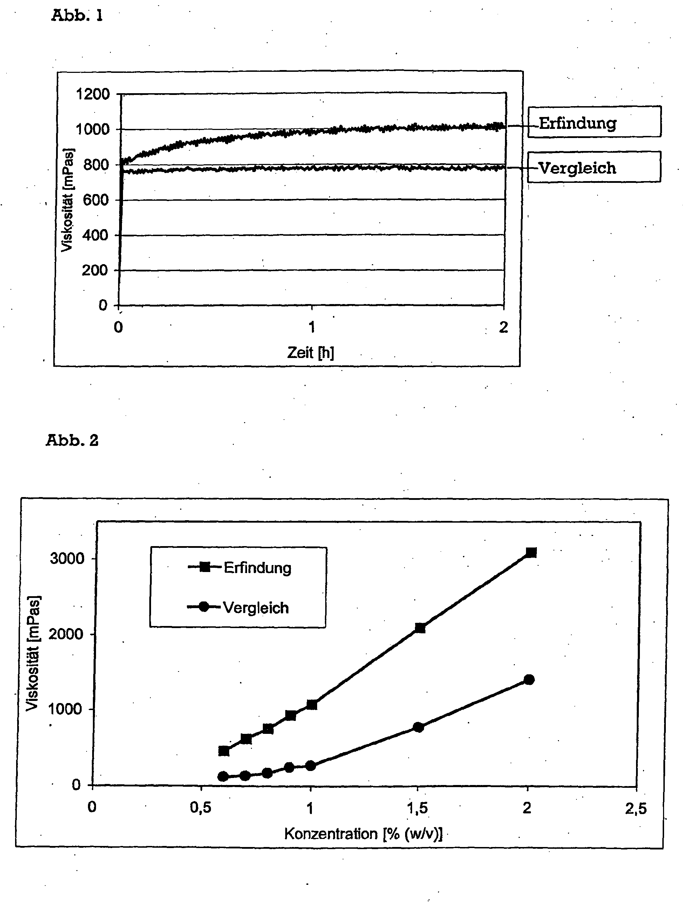

Die Ergebnisse dieses Beispiels sind in Abbildung 1 wiedergegeben. Im Vergleich zur Referenzprobe hat sich die Viskosität der Enzym-behandelten Probe innerhalb 2 Stunden um ca. 30 % erhöht.

### Beispiel 2: Enzymatische Modifizierung von Skleroglukan

Zu 100 mL destillierten Wassers wurde 1 g Skleroglukan (Actigum CS6, Degussa AG) gegeben und mit einem Propellerrüher 24 Stunden lang bei 20 °C gerührt. Anschließend wurde die Lösung auf 37 °C erwärmt und 1 mL einer 2 mg/mL destillierten Wassers an 1,3-β-Glukanase (Glucanex, Novozymes) enthaltenden Enzymlösung zugesetzt. Diese Lösung wurde für 3 Stunden bei 37 °C unter konstantem Rühren gehalten und dann in 1 000 mL reinen Alkohols (VWR Nr. 100943) gegeben. Mit Hilfe eines Filtersiebs (Maschenweite 70 µm) wurde dann das unlösliche Präzipitat von der Lösung abgetrennt und der abgetrennte Niederschlag bei 20 °C getrocknet sowie mit Hilfe einer Mühle pulverisiert.

Als Vergleich diente eine entsprechend hergestellte Probe, bei der anstelle der Enzymlösung 1 mL destillierten Wassers zugesetzt wurde. Die Viskosität der Enzym-behandelten erfindungsgemäßen Probe und der Referenzprobe wurden mit Hilfe eines Thermo Haake-Viskosimeters (Rotovisco C1) bei 20 ° C und einer Scherrate von 10/Sekunde bestimmt.

Abbildung 2 zeigt, dass die Viskosität des erfindungsgemäß modifizierten Skleroglukans annähernd doppelt so hoch ist wie die der unter vergleichbaren Bedingungen behandelten Referenzprobe.

### Beispiel 3: Enzymatische Behandlung eines unlöslichen Mycels

Zu 100 mL destillierten Wassers wurde 1 g Skleroglukan (Actigum CS6, Degussa AG) gegeben und mit einem Schraubenrührer 24 Stunden lang bei 20 °C gerührt. Dann wurde für 30 Minuten bei 1 500 rcf zentrifugiert, die Lösung abgetrennt und zum Bodensatz wurden 500 mL destillierten Wassers gegeben. Die daraus resultierende Suspension wurde mit einem Magnetrührer für 30 Minuten gerührt und die genannten Schritte (Zentrifugieren, Überstand entfernen, Wiederaufnehmen und Rühren der Suspension) fünfmal wiederholt. Nach dem letzten Zentrifugationsschritt wurde die Lösung entfernt und der Rückstand bei - 20 °C eingefroren. Dann wurde der gefrorene Rückstand bei 0,01 hPa für 24 Stunden gefriergetrocknet und anschließend 50 mg davon in 10 mL destillierten Wassers suspensiert. Zu dieser Suspension wurde 1 mL einer 2 mg/mL destillierten Wassers an 1,3-β-Glucanase (Glucanex, Novozymes) enthaltenen Enzymlösung gegeben und die erhaltene Lösung in ein Thermo Haake-Viskosimeter (Rotovisco C1) bei 37 °C und einer Schergeschwindigkeit von 10/Sekunde gegeben. Anschließend wurde die Messung gestartet.

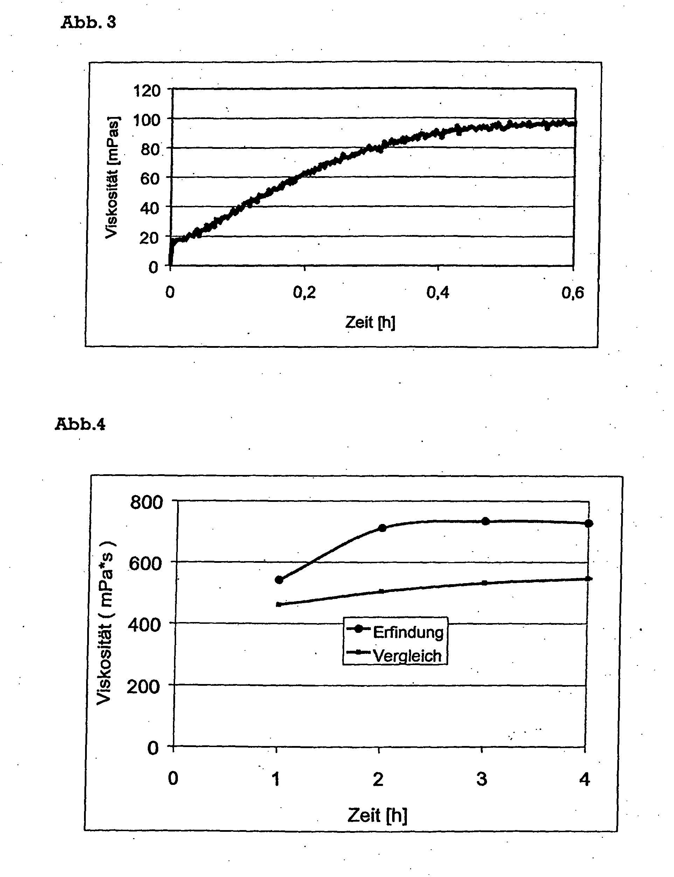

Abbildung 3 zeigt die mehr als zehnfache Viskositätszunahme, bei der gemäß Erfindung enzymbehandelten Mycel-Suspension, was darauf hindeutet, dass während der enzymatischen Behandlung das Polysaccharid vom unlöslichen Mycel befreit und im Wasser gelöst wurde. Gleichzeitig konnte eine Abnahme an unlöslichen Mycel-Partikeln beobachtet werden.

### Beispiel 4: Enzymatische Behandlung einer Fermentationsbrühe von Sclerotium

Zu 2080 g destillierten Wassers wurden 520 g einer Skleroglukan-Brühe (Degussa AG) gegeben und mit einem Hochscherrührer für 2 Stunden bei 25 °C gerührt, dann der pH der Lösung auf Werte zwischen 5,2 und 5,4 mit Hilfe einer 10 %igen NaOH-Lösung eingestellt und 2,6 g eines Enzym-Pulvers (Safizym GP, Saf-isis) in die Lösung gegeben und diese in Portionen zu 200 g aufgeteilt. Die Einzelportionen wurden anschließend für eine Dauer zwischen 1 und 6 Stunden bei 37 °C in einen Orbitalschüttler gegeben. Nach jeweils einer Stunde wurden einzelne Portionen aus dem Schüttler genommen, die Lösungen abgetrennt und die Viskosität mit einem Brookfield-Rheometer (LVTD, 30 rpm) bestimmt.

Abbildung 4 zeigt das Ergebnis der erfindungsgemäßen enzymatischen Behandlung der Fermentationsbrühe:
Innerhalb 2 bis 4 Stunden der Behandlungsdauer wurde das Polysaccharid vom unlöslichen Mycel befreit und gelöst, die Viskosität der Lösung nahm zu und erreichte einen um 40 % höheren Viskositätswert als die Referenzproben, die unter den vergleichbaren Bedingungen hergestellt wurden, allerdings ohne Enzymzusatz.

### Beispiel 5: Herstellung eines gereinigten Skleroglukans

150 g Skleroglukan (Actigum CS6, Degussa AG) wurden in 20 I destillierten Wassers unter Rühren mit einem Hochleistungsrührer bei 80 °C 2 Stunden lang gelöst. Dann wurde die Lösung auf 37 °C abgekühlt und 24 g Safizym CP (Saf-isis) zugegeben. Diese Suspension wurde bei 37 °C für 2,5 Stunden gerührt und anschließend die Lösung erneut auf 80 °C erhitzt. Anschließend wurden 500 g Filterende (FloM, CECA) zugegeben und die Suspension mit einer Filterpresse (Eurofiltec) filtriert. Das Filtrat wurde in 40 Liter 80 %igen Ethanol gegeben und das erhaltene Koagulat mit einem Filtersieb (Maschenweite 70 µm) filtriert. Abschließend wurde das Koagulat in einem Ofen bei 60 °C getrocknet und mit einem Gridgrinder (Retsch) vermahlen.

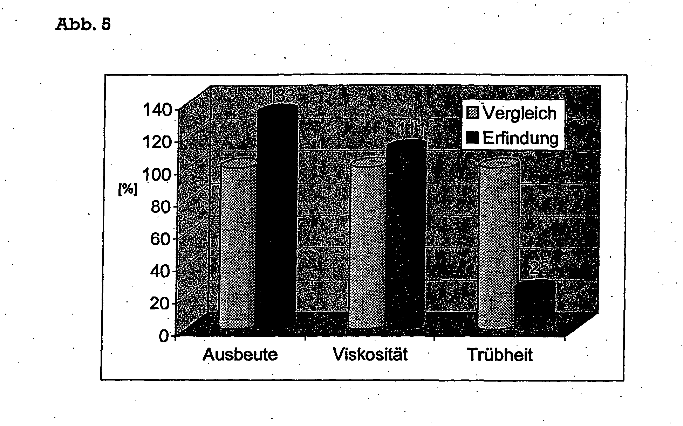

Als Vergleich diente eine Referenzprobe, die unter den identischen Bedingungen, allerdings ohne Enzymzusatz hergestellt wurde. Ein Vergleich dieser Referenzprobe und der erfindungsgemäß Enzym-behandelten Skleroglukan-Probe zeigt (Abbildung 5), dass die Ausbeute mit dem erfindungsgemäßen Verfahren nach dem Filtrationsschritt um ca. 30 % im Vergleich zur Referenzprobe höher ausfiel, wobei die Viskosität des filtrierten Skleroglukans zusätzlich 10 % höher war. Schließlich war die Trübheit der erfindungsgemäßen Probe im Vergleich zur Referenz um den Faktor 4 geringer.

## Patentansprüche

1. Verfahren zur Herstellung eines β-1,3-Glukans, **dadurch gekennzeichnet, dass** eine Glukan-haltige Matrix in Wasser als Lösemittel gerührt und anschließend bei Temperaturen zwischen 15 und 60 °C sowie bei einem pH-Wert zwischen 4,0 und 10,0 mit einem Protein mit β(1,3)-Glucanase-Aktivität behandelt wird.

2. Verfahren nach Anspruch 1, **dadurch gekennzeichnet, dass** das β-1,3-Glukan β(1,6)-Glucose-Seitenketten aufweist.

3. Verfahren nach einem der Ansprüche 1 oder 2, **dadurch gekennzeichnet, dass** es sich beim Protein um β(1,3)-Glucanase handelt.

4. Verfahren nach einem der Ansprüche 1 bis 3, **dadurch gekennzeichnet, dass** das Protein eine β(1,4)-Glucanase-Aktivität aufweist.

5. Verfahren nach einem der Ansprüche 1 bis 4, **dadurch gekennzeichnet, dass** die Konzentration des Proteins mit β(1,3)-Glucanase-Aktivität 0,001 bis 3,0 Gew.-%, insbesondere 0,01 bis 1,0 Gew.-% und besonders bevorzugt 0,1 bis 0,5 Gew.-%, jeweils bezogen auf die Reaktionsmischung, beträgt.

6. Verfahren nach einem der Ansprüche 1 bis 5, **dadurch gekennzeichnet, dass** es bei Temperaturen zwischen 20 und 40 °C und besonders bevorzugt bei Raumtemperatur durchgeführt wird.

7. Verfahren nach einem der Ansprüche 1 bis 6, **dadurch gekennzeichnet, dass** es sich bei der Glukan-haltigen Matrix um eine Fermentationsbrühe, ein Kulturmedium, eine Suspension oder ein Mycel, ein Hydrokolloid oder eine Pulver-Zubereitung mit einem Lösemittel-Anteil von 20 bis 99,9 Gew.-% und insbesondere von 50 bis 99 Gew.-%, jeweils bezogen auf den Feststoff-Gehalt, handelt.

8. Verfahren nach einem der Ansprüche 1 bis 7, **dadurch gekennzeichnet, dass** die Fermentationsbrühe oder das Kulturmedium ungelöste Feststoffe, Zellbestandteile und/oder Zellbruchstücke enthält.

9. Verfahren nach einem der Ansprüche 1 bis 8, **dadurch gekennzeichnet, dass** das Mycel, das Hydrokolloid oder die Pulver-Zubereitung als wässrige Lösung eingesetzt werden.

10. Verfahren nach einem der Ansprüche 1 bis 9, **dadurch gekennzeichnet, dass** die Matrix als Lösemittel Wasser enthält.

11. Verfahren nach einem der Ansprüche 1 bis 10, **dadurch gekennzeichnet, dass** der pH-Wert der Matrices zwischen 5,0 und 7,0 liegt.

12. Verfahren nach einem der Ansprüche 1 bis 11, **dadurch gekennzeichnet, dass** die Dauer der enzymatischen Behandlung 15 Minuten bis 24 Stunden und insbesondere 1 bis 6 Stunden beträgt.

13. Verfahren nach einem der Ansprüche 1 bis 12, **dadurch gekennzeichnet, dass** es kontinuierlich durchgeführt wird.

14. Verfahren nach einem der Ansprüche 1 bis 13, **dadurch gekennzeichnet, dass** die Glukan-haltige Matrix nach erfolgter enzymatischer Behandlung einer Wärmebehandlung bei Temperaturen zwischen 70 und 150 °C und insbesondere zwischen 80 und 140 °C unterzogen wird.

15. Verfahren nach Anspruch 14, **dadurch gekennzeichnet, dass** die Wärmebehandlung 1 bis 60 Minuten und insbesondere 2 bis 30 Minuten lang durchgeführt wird.

16. Verfahren nach einem der Ansprüche 1 bis 15, **dadurch gekennzeichnet, dass** die Glukan-haltigen Matrices abschließend einer Filtration und/oder Zentrifugation unterzogen werden.

17. Verfahren nach einem der Ansprüche 1 bis 16, **dadurch gekennzeichnet, dass** das Glukan abgetrennt wird, vorzugsweise durch Evaporation, Gefriertrocknung oder Ausfällung.

18. β-1,3-Glukan erhältlich mit einem Verfahren nach einem der Ansprüche 1 bis 17.

19. β-1,3-Glukan nach Anspruch 18, **dadurch gekennzeichnet, dass** es eine verbesserte Kaltwasserlöslichkeit und/oder verringerte Anteile an unlöslichen Bestandteilen und/oder eine gesteigerte Viskosität und/oder eine verringerte Trübheit in wässrigen Lösungen und/oder eine verbesserte Filtrierbarkeit aufweist.

20. Feste Formulierung enthaltend mind. 90 bis 99,9 Gew.-% eines unbehandelten β-1,3-Glukans, 0,005 bis 0,1 Gew.-% eines Proteins mit β(1,3)-Glucanase-Aktivität sowie 0 bis 10 Gew.-% mind. eines weiteren Inhaltsstoffes, wie Füllstoffe, inerte Verdünnungsmittel oder ein Mycel.

21. Verwendung eines nach den Ansprüchen 1 bis 19 hergestellten β-1,3-Glukans für kosmetische Anwendungen und/oder in der Körper- und Gesundheitspflege und/oder in der Nahrungs- und Lebensmittelindustrie und/oder in der Erdölförderung.

## Claims

1. Process for preparing a β-1,3-glucan, **characterized in that** a glucan-containing matrix is stirred in water as solvent and subsequently treated, at temperatures of between 15 and 60°C and at a pH of between 4.0 and 10.0, with a protein possessing β(1,3)-glucanase activity.

2. Process according to Claim 1, **characterized in that** the β-1,3-glucan possesses β(1,6)-glucose side chains.

3. Process according to one of Claims 1 and 2, **characterized in that** the protein is β(1,3)-glucanase.

4. Process according to one of Claims 1 to 3, **characterized in that** the protein possesses β(1,4)-glucanase activity.

5. Process according to one of Claims 1 to 4, **characterized in that** the concentration of the protein possessing β(1,3)-glucanase activity is from 0.001 to 3.0% by weight, in particular from 0.01 to 1.0% by weight, and particularly preferably from 0.1 to 0.5% by weight, in each case based on the reaction mixture.

6. Process according to one of Claims 1 to 5, **characterized in that** it is carried out at temperatures of between 20 and 40°C and, particularly preferably, at room temperature.

7. Process according to one of Claims 1 to 6, **characterized in that** the glucan-containing matrix is a fermentation broth, a culture medium, a suspension or a mycelium, a hydrocolloid or a powder preparation containing a solvent proportion of from 20 to 99.9% by weight and, in particular, of from 50 to 99% by weight, in each case based on the solids content.

8. Process according to one of Claims 1 to 7, **characterized in that** the fermentation broth or the culture medium contains undissolved solids, cell constituents and/or cell fragments.

9. Process according to one of Claims 1 to 8, **characterized in that** the mycelium, the hydrocolloid or the powder preparation is employed as an aqueous solution.

10. Process according to one of Claims 1 to 9, **characterized in that** the matrix contains water as solvent.

11. Process according to one of Claims 1 to 10, **characterized in that** the pH of the matrices is between 5.0 and 7.0.

12. Process according to one of Claims 1 to 11, **characterized in that** the duration of the enzymic treatment is from 15 minutes to 24 hours and, in particular, from 1 to 6 hours.

13. Process according to one of Claims 1 to 12, **characterized in that** it is carried out continuously.

14. Process according to one of Claims 1 to 13, **characterized in that** after it has been treated enzymically, the glucan-containing matrix is subjected to a heat treatment at temperatures of between 70 and 150°C and, in particular, of between 80 and 140°C.

15. Process according to Claim 14, **characterized in that** the heat treatment is carried out for from 1 to 60 minutes and, in particular, for from 2 to 30 minutes.

16. Process according to one of Claims 1 to 15, **characterized in that** the glucan-containing matrices are finally subjected to a filtration and/or centrifugation.

17. Process according to one of Claims 1 to 16, **characterized in that** the glucan is separated off, preferably by means of evaporation, freeze drying or precipitation.

18. β-1,3-Glucan, obtainable using a process according to one of Claims 1 to 17.

19. β-1,3-Glucan according to Claim 18, **characterized in that** it exhibits improved solubility in cold water and/or reduced proportions of insoluble constituents and/or an increased viscosity and/or reduced turbidity in aqueous solutions and/or improved filterability.

20. Solid formulation comprising at least from 90 to 99.9% by weight of an untreated β-1,3-glucan, from 0.005 to 0.1% by weight of a protein possessing β(1,3)-glucanase activity and from 0 to 10% by weight of at least one further constituent, such as fillers, inert diluents or a mycelium.

21. Use of a β-1,3-glucan which has been prepared according to Claims 1 to 19 for cosmetic applications and/or in body care and health care and/or in the foodstuffs industry and/or in oil production.

## Revendications

1. Procédé de préparation d'un β-1,3-glucane, **caractérisé en ce qu'**une matrice contenant du glucane est agitée dans l'eau comme solvant, et ensuite traitée à une température allant de 15 à 60 °C, ainsi qu'à un pH allant de 4,0 à 10,0, avec une protéine à activité de β(1,3)-glucanase.

2. Procédé selon la revendication 1, **caractérisé en ce que** le β-1,3-glucane présente des chaînes latérales β-1,6-glucose.

3. Procédé selon la revendication 1 ou 2, **caractérisé en ce que** la protéine consiste en une β(1,3)-glucanase.

4. Procédé selon l'une quelconque des revendications 1 à 3, **caractérisé en ce que** la protéine présente une activité de β(1,4)-glucanase.

5. Procédé selon l'une quelconque des revendications 1 à 4, **caractérisé en ce que** la concentration de la protéine avec activité de β(1,3)-glucanase se situe dans l'intervalle allant de 0,001 à 3,0 % en poids, en particulier de 0,01 à 1,0 % en poids et de manière particulièrement préférée, de 0,1 à 0,5 % en poids, chaque fois sur base du mélange réactionnel.

6. Procédé selon l'une quelconque des revendications 1 à 5, **caractérisé en ce qu'**il est réalisé à une température allant de 20 à 40 °C et de manière particulièrement préférée, à température ambiante.

7. Procédé selon l'une quelconque des revendications 1 à 6, **caractérisé en ce que** la matrice contenant un glucane consiste en un bouillon de fermentation, un milieu de culture, une suspension ou un mycélium, un hydrocolloïde ou une composition pulvérulente avec une proportion de solvant allant de 20 à 99,9 % en poids et en particulier, de 50 à 99 % en poids, chaque fois sur base de la teneur en matières solides.

8. Procédé selon l'une quelconque des revendications 1 à 7, **caractérisé en ce que** le bouillon de fermentation ou le milieu de culture contient des matières solides, constituants cellulaires et/ou débris cellulaires non dissous.

9. Procédé selon l'une quelconque des revendications 1 à 8, **caractérisé en ce que** le mycélium, l'hydrocolloïde ou la composition pulvérulente est mis en oeuvre sous forme d'une solution aqueuse.

10. Procédé selon l'une quelconque des revendications 1 à 9, **caractérisé en ce que** la matrice contient de l'eau comme solvant.

11. Procédé selon l'une quelconque des revendications 1 à 10, **caractérisé en ce que** le pH de la matrice se situe dans l'intervalle allant de 5,0 à 7,0.

12. Procédé selon l'une quelconque des revendications 1 à 11, **caractérisé en ce que** la durée du traitement enzymatique se situe dans l'intervalle allant de 15 minutes à 24 heures, en particulier de 1 à 6 heures.

13. Procédé selon l'une quelconque des revendications 1 à 12, **caractérisé en ce qu'**il est réalisé de manière continue.

14. Procédé selon l'une quelconque des revendications 1 à 13, **caractérisé en ce que** la matrice contenant un glucane est soumise après le traitement enzymatique, à un traitement thermique à des températures allant de 70 à 150 °C et en particulier, de 80 à 140 °C.

15. Procédé selon la revendication 14, **caractérisé en ce que** le traitement thermique est réalisé pendant 1 à 60 minutes, en particulier 2 à 30 minutes.

16. Procédé selon l'une quelconque des revendications 1 à 15, **caractérisé en ce que** la matrice contenant un glucane est soumise ensuite, à une filtration et/ou une centrifugation.

17. Procédé selon l'une quelconque des revendications 1 à 16, **caractérisé en ce que** le glucane est séparé, de préférence par évaporation, lyophilisation ou précipitation.

18. β-1,3-Glucane obtenu par un procédé selon l'une quelconque des revendications 1 à 17.

19. β-1,3-Glucane selon la revendication 18, **caractérisé en ce qu'**il présente une solubilité dans l'eau froide améliorée et/ou une proportion diminuée de constituants insolubles et/ou une viscosité augmentée et/ou un trouble diminué en solutions aqueuses et/ou une aptitude à la filtration améliorée.

20. Formulation solide contenant au moins 90 à 99,9 % en poids d'un β-1,3-glucane non traité, 0,005 à 0,1 % en poids d'une protéine à activité β(1,3)-glucanase ainsi que 0 à 10 % en poids d'au moins un autre constituant, comme des charges, des agents de dilution inertes ou un mycélium.

21. Utilisation d'un β-1,3-glucane préparé selon l'une quelconque des revendications 1 à 19, pour des applications cosmétiques et/ou dans les soins du corps et de santé et/ou dans l'industrie alimentaire et/ou dans l'extraction du pétrole.
